# EUROPEAN PATENT APPLICATION

(11) **EP 2 862 578 A1**
(43) Date of publication of application: **22.04.2015**
(21) Application number: 14189464.2
(22) Date of filing: 20.10.2014
(51) Int. Cl.: A61K 38/35, A61P 25/00, A61K 9/00

(54) **Parenteral pharmaceutical composition containing cosyntropin**

(30) Priority: 21.10.2013 US 201361893745 P
(71) Applicant: EMS S.A., 13186-901 Sao Paulo - SP (BR)
(72) Inventor: Santus, Giancarlos, Milano (IT); Khater Covesi, Leticia, Sao Paulo (BR); Donadoni, Luca, Alzano Lombardo (IT)
(74) Representative: Cabinet Plasseraud

(57) **Abstract**

The present invention describes novel and improved parenteral depot pharmaceutical compositions containing cosyntropin (tetracosactide), for the treatment of infantile spasm.

## Description

### Field of the invention

This invention relates to novel and improved parenteral dosage forms and pharmaceutical compositions containing a synthetic polypeptide, for the treatment of infantile spasm. In particular the present invention relates to a preservative-free dosage form containing cosyntropin (tetracosactide) in prefilled syringes with improved safety and enhanced easiness of use, a process for obtaining said dosage form, its use and a preservative-free pharmaceutical composition containing cosyntropine (tetracosactide)

### Background

Corticotropin, (adrenocorticotropic hormone - ACTH), is a polypeptide tropic hormone produced and secreted by anterior pituarity gland, also called adenohypophysis, and consists of 39 amino acids. Its principal effects are increased production and release of corticosteroids.

Cosyntropin (tetracosactide) is a synthetic polypeptide identical to the first 24 amino acids of human adrenocorticotropic hormone (ACTH). The relationship between the sequence of the amino acids of ACTH and of cosyntropin is therefore the following (the common fragment of the sequence is between the brackets in bold):
**[H-Ser-Tyr-Ser-Met-Glu-His-Phe-Arg-Trp-Gly-Lys-Pro-Val-Gly-Lys-Lys-Arg-Arg-Pro-Val-Lys-Val-Tyr-Pro]**Asp-Ala-Gly-Glu-Asp-Gln-Ser-Ala-Glu-Ala-Phe-Pro-Leu-Glu-Phe.

Cosyntropin (tetracosactide) is used in clinical medicine for diagnostic and therapeutic purposes.

The diagnostic use is for the investigation of adrenocortical insufficiency and includes the diagnose of adrenal gland problems such as Addison's disease, insufficiency due to corticosteroid use and pituitary tumor.

The therapeutic use is normally only for short-term therapy in conditions for which glucocorticoids are indicated in principle, for example, in ulcerative colitis and Crohn's disease, juvenile rheumatoid arthritis, or as adjunct therapy in patients with rheumatoid arthritis and osteoarthrosis. It may be particularly useful in patients unable to tolerate oral glucocorticoid therapy or in patients where normal therapeutic doses of glucocorticoids have been ineffective.

EP 1878454 describes an antihemorragic treatment kit useful in emergency situations that comprises tetracosactide hexaacetate and an auto-injector for automatically injecting said drug into a patient.

Other uses are for the treatments of inflammatory diseases, neurodegenerative disease and infantile spasm.

An infantile spasm (IS) is a specific type of seizure seen in an epilepsy syndrome of infancy and childhood known as West Syndrome. West Syndrome is characterized by infantile spasms, developmental regression, and a specific pattern on electroencephalography (EEG) testing called hypsarrhythmia (chaotic brain waves). The onset of infantile spasms is usually in the first year of life, typically between 4-8 months. The seizures primarily consist of a sudden bending forward of the body with stiffening of the arms and legs; some children arch their backs as they extend their arms and legs. Spasms tend to occur upon awakening or after feeding, and often occur in clusters of up to 100 spasms at a time. Infants may have dozens of clusters and several hundred spasms per day. Infantile spasms usually stop by age five, but may be replaced by other seizure types. Many underlying disorders, such as birth injury, metabolic disorders, and genetic disorders can give rise to spasms, making it important to identify the underlying cause.

For the treatment of infantile spasm is presently used Acthar® Gel a long-lasting animal product. Acthar® Gel is created from pig pituitary glands and it is currently an extremely expensive pharmaceutical product. A limitation to its use, apart from the very high cost, is the possibility of allergic reaction to pig derived proteins.

An alternative product for the treatment of infantile spasm is Synacthen® depot, a suspension in which the active substance cosyntropin (tetracosactide) is adsorbed onto an inorganic zinc complex.

Pharmaceutical stable long acting preparations with zinc salts are reported in US 3228839 and US 3243345.

The inventors surprisingly found that it is possible to prepare stable and safe cosyntropin (tetracosactide) depot preservative-free dosage forms and pharmaceutical compositions in prefilled syringes for the treatment in particular of infantile spasm.

### Summary of the invention

One embodiment of the present invention is a preservative-free depot dosage form of a synthetic polypeptide wherein the said synthetic polypeptide is identical to the first 24 amino acids of human adrenocorticotropic hormone (ACTH), preferably said polypeptide is cosyntropin (tetracosactide), more preferably tetracosactide hexaacetate.

A second embodiment of the present invention refers to a pharmaceutical composition comprising a synthetic polypeptide wherein the said synthetic polypeptide is identical to the first 24 amino acids of human adrenocorticotropic hormone (ACTH). Preferably, the pharmaceutical composition is a preservative-free depot pharmaceutical composition comprising cosyntropin (tetracosactide), and also preferably the pharmaceutical composition is prolonged released.

The preservative-free depot dosage form or the pharmaceutical composition of the present invention is preferably a suspension with particles whose dimension is less than 50 µm for a single particle.

The preservative-free depot dosage form or the pharmaceutical composition of the present invention is preferably prefilled in a single unit container, preferably in syringes.

A third embodiment of the present invention is a process for preparing the preservative free depot dosage form or the pharmaceutical composition of the present invention containing a synthetic polypeptide, preferably cosyntropin (tetracosactide), in a depot form, said process comprising the steps of:
(i) preparing a solution (A) by dissolving zinc chloride in water for injection, and dissolving a buffering agent, preferably a phosphate buffer in water for injection, adding the zinc chloride solution to the buffering agent solution and filtering solution (A) through a membrane;
(ii) preparing a solution (B) by dissolving an excipient, preferably sodium chloride in sodium hydroxide in water and filtering solution (B) through a membrane;
(iii) preparing a solution (C) by dissolving the synthetic polypeptide, preferably cosyntropin (tetracosactide) in water for injection and filtering the solution through a membrane,
(iv) mixing solution (A) and (C);
(v) adding to the resultant solution (iv) the solution (B), while stirring;
(vi) adjusting the pH value of the final solution (v) to 7.0 - 10.0 with sodium hydroxide or hydrochloric acid;
(vii) filling glass syringes with the solution (vi).

A fourth embodiment would be the preservative free depot dosage form or the pharmaceutical composition of the present invention for its use to prepare a medicament for the treatment or prophylaxis of diseases for which corticosteroids are indicated, preferably for the treatment or prophylaxis of infantile spasm.

### Description of the invention

The present invention relates particularly to novel and improved pharmaceutical compositions containing cosyntropin (tetracosactide), for the treatment of infantile spasm. In particular the present invention relates to preservative-free parenteral pharmaceutical compositions with improved safety and enhanced easiness of use.

Depot pharmaceutical composition of cosyntropin (tetracosactide) zinc complex Synacthen® is presently available in ampoules and it contains benzyl alcohol as antimicrobial preservative of the pharmaceutical composition. Other components of Synacthen® depot are zinc chloride, disodium phosphate dodecahydrate, sodium chloride, sodium hydroxide, hydrochloric acid and water for injections.

Benzyl alcohol is used in a wide variety of pharmaceutical compositions in concentrations from 0.5 % up to 3% as antimicrobial preservative. Benzyl alcohol is normally oxidized rapidly to benzoic acid which is further metabolized in the liver by conjugation with glycine to form hyppuric acid, which is excreted in the urine.

High concentrations can result in toxic effects including respiratory failure, vasodilation, hypotension, convulsions, and paralysis. Newborns, especially if critically ill, may not metabolize benzyl alcohol as readily as adults. Reports in the early 1980s of sixteen neonatal deaths associated with the use of saline flush solutions containing benzyl alcohol preservative led to recommendations to avoid its use in neonate [http://www.cdc.sov/MMWR/preview/mmwrhtml/00001109.htm].

The inventors found now that it is possible to have stable and safe pharmaceutical composition free of benzyl alcohol. Due to potential toxicity of benzyl alcohol this pharmaceutical composition is particularly advantageous for the treatment of infantile spasms in newborn babies.

The process for preparing the preservative free depot dosage form or the pharmaceutical composition of the invention involves mixing three sterile solutions (A), (B) and (C), comprising excipients and a synthetic polypeptide, as follows.

Excipients for this pharmaceutical composition may be selected from buffering agents, acidifying and alkalinizing agents. Preferred buffering agents are, for example, disodium hydrogen phosphate dodecahydrate, potassium metaphosphate or potassium phosphate. Even more preferred buffering agent is disodium hydrogen phosphate dodecahydrate. Acidifying agents may be selected from a group consisting of organic and inorganic acids such as citric acid, acetic acid, hydrochloric acid. Preferably, the acidifying agent is hydrochloric acid. Alkalinizing agents may be selected from a group consisting of organic and inorganic bases such as tromethamine, sodium hydroxide and potassium hydroxide. The most preferred alkalinizing agent is sodium hydroxide.

Solution (A) is prepared by dissolving zinc chloride in water for injection and dissolving a buffering agent, preferably disodium hydrogen phosphate dodecahydrate in water for injection at a temperature that facilitate the dissolution. For this invention, the temperature to dissolve the disodium hydrogen phosphate dodecahydrate may vary between 40 °C and 80 °C. Preferably the temperature to dissolve the disodium hydrogen phosphate dodecahydrate in water for injection is 60 °C.

The zinc chloride solution is added to the disodium hydrogen phosphate dodecahydrate solution at room temperature, and a white precipitate of zinc hydroxide is immediately formed. Hydrogen chloride in a concentration between 0.5 and 1.5 M is added to dissolve the precipitate until a solution is obtained. The preferred concentration for this invention is 1 M of hydrogen chloride. The solution has a pH value between 1.0 and 3.0. Preferably between 2.0 and 2.2.

Finally, solution (A) obtained as described above is filtered through a membrane. Preferably the membrane is a 0.2 µm PVDF membrane.

Solution (B) is prepared by dissolving an excipient, preferably sodium chloride in a strong base, preferably sodium hydroxide in water. The sodium hydroxide should preferably be in a concentration that varies between 0.5 and 1.5 M. Preferably the concentration is 1 M of sodium hydroxide. The resultant solution must be filtered through a membrane. Preferably the membrane is a 0.2 µm PVDF membrane. Solution (C) is prepared by dissolving a synthetic polypeptide in water for injection. The final pH value for this solution (C) should preferably be adjusted to a range between 2.0 and 3.0. Preferably the pH for the final solution (C) has a value between 2.5 and 2.8. The pH is preferably adjusted with hydrogen chloride.

Solution (C) should also preferably be filtered through a PVDF membrane. Preferably the membrane is a 0.2 µm PVDF membrane.

Preferably, the final suspension solution is obtained by mixing solutions (A), (B) and (C) as described below:
1. Mixing solution (A) and (C),
2. While stirring the resultant solution, adding solution (B).
3. Adjusting the pH value between 7.0 and 10.0 by adding a strong acid such as hydrochloric acid or a strong base such as sodium hydroxide.
4. A zinc complex is formed with the adsorption of the cosyntropin (tetracosactide) at the zinc hydroxide precipitate.
5. Adding water for injection up to final volume.

Preferably, the pH value of the final suspension in step 3 above for this invention is between 7.8 and 9.2.

The inventors surprisingly found that a very convenient way of delivering cosyntropin (tetracosactide) in a depot dosage form or pharmaceutical composition is through parenteral administration, preferably by preparing a suspension and filling it under nitrogen in prefilled syringes. According to one embodiment, the preservative-free dosage form of the invention is a depot parenteral pharmaceutical composition, preferably with zinc phosphate. Parenteral injection may be, for example, intravenous, subcutaneous or intramuscular administration. Most preferably the administration of the parenteral injection is intramuscular.

Prefilled syringes are used in a variety of areas, including medical test and therapeutic use. Prefilled syringe has been the preferred primary container for many parenteral delivery systems given the inherent benefits of convenience, safe handling and lower risk of contamination compared to vials. The need for precise dosing is met with prefilled syringes, whereas vials add a level of manipulation to the administration process. This is of particular relevance for peptides and proteins. The currently existing market of prefilled syringes is in the US around US$1-2 billion with a growth rate expected of 10% annually.

Prefilled syringes typically have a syringe body or syringe barrel and a plunger. The plunger head seals to the inner surface of the barrel forming a sealed cavity or chamber that holds a fluid, such as the therapeutic substance in an appropriate pharmaceutical composition. The plunger includes a shaft coupled at one end to the plunger head, and to the other end, the shaft is coupled to a plunger top or disc, sized to facilitate engagement by a user's finger or thumb. Pushing the plunger disc forces the plunger head toward dispensing opening located through the syringe body resulting in the dispensing or ejection of fluid from the syringe body.

Prefilled syringes can be of plastic material (such as cyclic olefin polymers and copolymers) or glass, preferably for injectable. Preferred prefilled syringes are in glass type 1. Glass is the material of choice because it is strong, chemically inert, dimensionally stable, and easy to sterilize. Further, it is transparent, which allows visible inspection of a dosage form before it is injected.

Needle options for prefilled syringes include staked-in (needle is manufactured as part of the syringe) and Luer cone or Luer Lock designs. Staked prefilled syringes are primarily used in medical and emergency situations (where treatment speed may be vital), whereas luered-prefills are mainly used for self-administration purposes. The preferred prefilled syringes of this invention have the needle separated in a sterile container in the package, to be inserted on the body of the syringe by the user.

Prefilled syringes are filled aseptically; sterile plungers are inserted, and individual units are packaged in blister packaging before shipping. To facilitate the filling process, sterile prefilled syringe barrels are usually shipped to filling facilities in nested carriers or tubs and then filled aseptically with automated fill-finish machinery.

The final suspension is then filled in glass syringes, preferably into ethylene oxide sterilized glass syringes kept under nitrogen atmosphere.

All the preparations of the present invention to be used in humans must be manufactured under aseptic conditions and under the protective atmosphere of nitrogen. All the solutions and the nitrogen used for the preparation of the suspensions are filtered through 0.2 µm filter.

The present invention will now be illustrated by the following examples. It is understood, however, that such examples are provided for illustration only, and the invention is not intended to be limited by the examples. The pharmaceutical compositions based on the system employed in the examples can be formed by any suitable method known in the art.

### Example 1. Pharmaceutical composition of cosyntropin (tetracosactide) zinc depot injection

| *Composition* | *mg*/*mL* |
|---|---|
| Tetracosactide hexaacetate | 1.00 |
| Disodium hydrogen phosphate dodecahydrate | 2.11 |
| Sodium chloride | 2.00 |
| Zinc chloride anhydrous | 5.21 |
| Water for injection | up to 1 mL |

### Preparation process

The preparation of tetracosactide hexaacetate depot suspension consists in the mixing of three sterile solutions A, B and C.

### Solution A

Dissolve 5.21 g of zinc chloride in 104.2 ml of water for injection.

Dissolve 2.11 g of disodium hydrogen phosphate dodecahydrate in 25 ml of water for injection at 60°C to facilitate the dissolution.

Add the zinc chloride solution to disodium hydrogen phosphate dodecahydrate solution at room temperature; immediately a white precipitate of zinc hydroxide is formed.

Add while stirring 1 M HCl to dissolve the precipitate until obtain a solution with a pH value between 2.0 and 2.2.

Finally add water for injection up to 250 ml and filter the solution through a 0.2 µm PVDF membrane.

### Solution B

Dissolve 2.0 g of sodium chloride in 82.64 g of 1 M NaOH and add water for injection up to 337.0 ml.

Filter the solution through a 0.2 µm PVDF membrane.

### Solution C

Dissolve 1.0 g of tetracosactide hexaacetate in 200 ml of water for injection and adjust the pH to a value between 2.5 and 2.8 with 1 M HCl (API is stable at acid pH). Filter the solution through a 0.2 µm PVDF membrane.

### Final suspension

Mix the solutions A and C. Add while stirring the solution B. Alkalizing cause the precipitate of zinc hydroxide; tetracosactide hexaacetate is adsorbed and there is the formation of a complex.

Adjust the pH value between 7.8 and 9.2 adding 1M HCl or 0.6M NaOH and add water for injection up to 1.0 liter.

### Filling of syringes

Share the suspension into ethylene oxide sterilized glass syringes kept under nitrogen atmosphere.

Seal using gamma ray sterilized rubber plunger stopper and tip cap.

*The preparation of solutions and final suspension is carried out in a grade B room while filling process is carried out in a grade A room. Preparation of solutions, filling of the final suspension is performed under protective atmosphere of nitrogen.

### Example 2. Technological characteristics of suspension

The preparation of example 1 has the following technological characteristics:
Appearance: Milky-white flocculent suspension which settles slowly but is readily resuspended.
Alkalinity: a pH value between 7.8 and 9.2, necessary for the complex stability.
Osmolarity: 280-310 mOsm. Isotonicity is important not to cause tissue damage.
Height of the sediment: between 10 to 20 mm. The test is performed by transferring 3.0 ml of shaken suspension into a cuvette 10x10 mm in cross section. After five hours at room temperature the height of sediment is measured.
Particle size: On examination under an optical microscope the particles are seen as amorphous particles or aggregates thereof. The average dimension of single particles is less than 50 µm.

### Example 3. Stability

The stability of the pharmaceutical composition reported in example 1 has been controlled with the HPLC method reported in the USP monograph cosyntropin as well in BP 2012 monograph for tetracosactide zinc injection.

Mobile phase: 365 mL acetonitrile, 10 mL glacial acetic acid and 10 g ammonium sulfate diluted with water to 2000 mL (pH 3.3)
Column: Nucleosil 120-C-18 4.6 mmx25 cm; 5υm packing
Detector UV: 280 nm
Column temperature 40 °C
Flow rate: 1.0 mL/min.
Injection volume: 50 µL
Run time: 50 min.

The preparation of example 1 has been put under stability for 1- 3 months at 4°C, 25 °C (room temperature) and 40 °C and compared with the preparation available on the market (Synacthen^{(R)} depot).

From the analytical degradation profile and the technological and microbiological controls it can be concluded that the preparation of example 1 has at least the same stability properties of commercial Synacthen^{(R)} depot.

The invention herein described is not limited to these embodiments and, those who have skills in the art will realize that any particular characteristic here introduced, should be only understood as something that was described to ease the comprehension and cannot be made without departing from the described inventive concept. The limiting characteristics of the subject of the present invention are related to the claims that are part of the present specification.

## Claims

1. A preservative-free dosage form comprising a synthetic polypeptide wherein the said synthetic polypeptide is identical to the first 24 amino acids of human adrenocorticotropic hormone (ACTH).

2. A preservative-free dosage form as defined in claim 1 wherein the said preservative-free dosage form is a depot parenteral pharmaceutical composition.

3. A preservative-free dosage form as defined in any of claims 1-2 wherein the said preservative-free dosage form is a depot parenteral pharmaceutical composition with zinc phosphate.

4. A preservative-free dosage form as defined in any of claims 1-3 wherein the synthetic polypeptide is cosyntropin (tetracosactide).

5. A preservative-free dosage form as defined in any of claims 1-4 wherein the preservative-free dosage form is prefilled in a single unit container, preferably in a prefilled syringe.

6. A preservative-free dosage form as defined in any of claims 1-5 wherein the prefilled syringe is of glass for injectable.

7. A process for the preparation of a preservative-free dosage form as defined in any of claims 1 to 6 containing a synthetic polypeptide, preferably cosyntropin (tetracosactide), in a depot form, said process comprising the steps of:
(i) preparing a solution (A) by dissolving zinc chloride in water for injection, and dissolving a buffering agent, preferably a phosphate buffer in water for injection, adding the zinc chloride solution to the buffering agent solution and filtering solution (A) through a membrane;
(ii) preparing a solution (B) by dissolving an excipient, preferably sodium chloride in sodium hydroxide in water and filtering solution (B) through a membrane;
(iii) preparing a solution (C) by dissolving the synthetic polypeptide, preferably cosyntropin (tetracosactide) in water for injection and filtering the solution through a membrane,
(iv) mixing solution (A) and (C);
(v) adding to the resultant solution (iv) the solution (B), while stirring;
(vi) adjusting the pH value of the final solution (v) to 7.0 - 10.0 with sodium hydroxide or hydrochloric acid;
(vii) filling glass syringes with the solution (vi).

8. The process as defined in claim 7 wherein the phosphate buffer in step (i) is disodium hydrogen phosphate dodecahydrate.

9. The process as defined in claim 8 wherein the disodium hydrogen phosphate dodecahydrate is dissolved at a temperature varying between 40 °C and 80 °C.

10. The process as defined in claim 9 wherein the temperature to dissolve the disodium hydrogen phosphate dodecahydrate is 60 °C.

11. The process as defined in claim 7 wherein the pH value range in step (vi) is preferably between 7.8-9.2.

12. The process as defined in claim 7 wherein the membrane used to the filter the solutions in steps (i), (ii) and (iii) is preferably a 0.2 µm PVDF membrane.

13. A pharmaceutical composition comprising a synthetic polypeptide wherein the said synthetic polypeptide is identical to the first 24 amino acids of human adrenocorticotropic hormone (ACTH).

14. The pharmaceutical composition as defined in claim 13 wherein the synthetic polypeptide is cosyntropin (tetracosactide).

15. The pharmaceutical composition as defined in any of claims 13-14 wherein the pharmaceutical composition is prefilled in a single unit container, preferably in a syringe of glass for injectable.

16. The pharmaceutical composition as defined in claims 13-15 wherein the said pharmaceutical composition is free of preservatives and is prolonged released.

17. The pharmaceutical composition as defined in any of claims 13-16 wherein the said pharmaceutical composition is a suspension with particles whose dimension is less than 50 µm for a single particle.

18. A preservative-free dosage form as defined in any of claims 1-6, for its use to prepare a medicament for the treatment or the prophylaxis of diseases for which corticosteroids are indicated.

19. A preservative-free dosage form as defined in claim 18, for its use to prepare a medicament for the treatment or the prophylaxis of infantile spasm.

20. A pharmaceutical composition as defined in any of claims 13 to 17 for its use to prepare a medicament for the treatment or the prophylaxis of diseases for which corticosteroids are indicated.

21. A pharmaceutical composition as defined in claim 20 for its use to prepare a medicament for the treatment or the prophylaxis of infantile spasm.
